# EUROPÄISCHE PATENTSCHRIFT

(11) **EP 3 458 650 B1**
(45) Veröffentlichungstag und Bekanntmachung des Hinweises auf die Patenterteilung: **27.11.2019**
(21) Anmeldenummer: 18727661.3
(22) Anmeldetag: 15.05.2018
(51) Int. Cl.: E03C 1/04, E03C 1/06, B05B 1/18, A61L 2/10, C02F 1/32

(54) **WASSERDESINFEKTIONSVERFAHREN UND WASSERZAPFSTELLENANORDNUNG DAFÜR**
WATER DISINFECTION METHOD AND WATER TAP CONNECTION ARRANGEMENT THEREFOR
PROCÉDÉ DE DÉSINFECTION DE L'EAU ET DISPOSITIF DE PRISE D'EAU ASSOCIÉ

(30) Priorität: 13.07.2017 DE 102017115743
(43) Veröffentlichungstag der Anmeldung: 27.03.2019
(73) Patentinhaber: Gravity Holding Ltd., Dubai (AE)
(72) Erfinder: HÖHNE, Bernd, 28832 Achim (DE)
(74) Vertreter: Hansen, Jochen
(86) Internationale Anmeldenummer: PCT/DE2018/100463
(87) Internationale Veröffentlichungsnummer: WO 2019/011369

(56) Entgegenhaltungen:
- WO-A2-2011/052986
- CN-B- 103 962 256
- CN-U- 203 695 256
- DE-A1- 10 157 355
- DE-U1- 29 614 998
- GB-A- 2 288 974
- US-A1- 2016 331 855

## Beschreibung

Die Erfindung betrifft ein Verfahren zur Desinfektion von Wasser mittels UV-Bestrahlung in Wasserversorgungsanlagen in Gebäuden, Schiffen oder dergleichen mit einer flexibel an einem Schlauch angeschlossenen Wasserzapfstelle, insbesondere Handbrause, und einer Wasserzapfstellenhalterung zum Aufnehmen der Wasserzapfstelle bei Nichtgebrauch, wobei die UV-Bestrahlung an der Wasserzapfstelle erfolgt. Ferner betrifft die Erfindung eine Wasserzapfstellenanordnung in Wasserversorgungsanlagen in Gebäuden, Schiffen oder dergleichen mit einer flexibel an einem Schlauch angeschlossenen Wasserzapfstelle, insbesondere Handbrause, und einer Wasserzapfstellenhalterung für das Aufnehmen der Wasserzapfstelle bei Nichtgebrauch, wobei Mittel zur UV-Bestrahlung des in der Wasserzapfstelle geführten Wassers vorgesehen sind.

Wasserversorgungsanlagen für Brauch- und Trinkwasser in Gebäuden und beispielsweise auch auf Schiffen sind dafür bekannt, dass Keime, insbesondere Legionellen, sich in ihnen so vermehren, dass sie immer wieder als Ursache für Krankheitsfälle zu beklagen sind. Nicht abtrocknendes Restwasser in Wasserzapfstellen bietet den Keimen Bedingungen, die ihre Vermehrung besonders begünstigt. So verunreinigtes Wasser wird dann insbesondere aus Duschköpfen in kleinsten Tropfen bis nebelartig abgegeben. Dieser Wassernebel kann leicht eingeatmet werden und birgt damit die Gefahr für Menschen die Atemgänge und die Lunge direkt zu infizieren. Die Infizierung von Lungen und Atemwegen mit Legionellen kann zu schweren Erkrankungen führen.

Eine häufig angewandte Gegenmaßnahme ist die Erhitzung der gesamten Rohrleitungssysteme durch hochtemperiertes Wasser und dessen kompletter Durchspülung mit frischem Wasser danach, um das System von alle Keimresten zu befreien. Weitere bekannte Gegenmaßnahmen sind zentrale Filterungen des Wassers oder die zentrale Desinfizierung mittels chemischer, elektrischer und auch lichttechnischer Verfahren, die auf ultraviolettem Licht (UV-Licht) basieren.

Nachteil der Durchspülung mit heißem Wasser ist, dass der Energieaufwand und der Wasserverbrauch dafür sehr hoch sind und die Maßnahme regelmäßig wiederholt werden muss. Trotzdem ist nur schwer sicherzustellen, dass alle besonders gefährdeten Komponenten wirkungsvoll desinfiziert sind.

Nachteil von zentralen Filtern und Desinfektionssystemen ist, dass auch wünschenswerte Mikroben und Wasserbestandteile dabei reduziert oder vernichtet werden können. Da Wasser in seiner ursprünglichen Form mit allen Bestandteilen ein natürliches Lebensmittel ist, ist eine zentrale Desinfektion nicht im Sinne aller Verbraucher.

Nachteilig ist, dass alle zentral ansetzenden Systeme zur Verhinderung schädlicher Keime, wie Legionellen, nicht gezielt an den Stellen des Wasserversorgungssystems ansetzen, die ein besonderes Gefährdungspotential besitzen, wie Zapfstellen und insbesondere Duschköpfe. Vielmehr muss die gesamte Wasserversorgung bei diesen zentralen Systemen desinfiziert werden, woraus ein hoher Aufwand entsteht und die besonderen Gefährdungsstellen, nämlich Zapfstellen und insbesondere Duschköpfe gleichwohl nicht ausreichend von einer unerwünschten Keimentwicklung ausgeschlossen sind. Gerade an Zapfstellen und insbesondere in Duschköpfen kann bei dort verbliebenem Restwasser bei Raumtemperatur und unter Lufteinfluss ein beschleunigtes Legionellenwachstum auftreten. Zudem können zentrale Systeme nicht verhindern, dass Keime an nicht zentralen Stellen durch eventuell fehlerhafte Komponenten oder andere Ursachen ins System eindringen, sich verteilen und sich dann an für sie günstigen Stellen vermehren.

Daher sind Anlagen zur UV-Bestrahlung in Wasserzapfstellen, insbesondere Brauseköpfen bekannt.

Die DE 101 57 355 A1 zeigt eine Ausgestaltung, bei der die UV-Bestrahlung in einer Brauchwasser führenden Leitung unmittelbar vor dem Duschkopf, beispielsweise im Brauseschlauch, erfolgt. Damit kann in der Leitung, jedoch nicht im Brausekopf verbleibendes Restwasser durch die UV-Bestrahlung desinfiziert werden.

Ein entsprechender Brausekopf ist aus der DE 196 39 802 A1 bekannt, bei dem im Brausekopf verbleibendes Restwasser mittels einer UV-Lampe auch nach dem Schließen der Wasserzapfstelle bestrahlt und somit entkeimt wird.

Aus der DE 197 36 636 A1 ist eine Einrichtung zum Entkeimen von Wasser in einer Wasserzapfstelle mittels UV-Bestrahlung bekannt, bei der zunächst die UV-Lampe aktiviert wird und erst anschließend der Wasserfluss aus der Wasserzapfstelle freigegeben wird. Damit soll eine Vorabentkeimung vor dem Beginn des Wasserauslaufs erreicht werden.

Ähnliche Anordnungen sind aus der CN 203695256 U sowie JP 2001334179 A oder der KR 1020080093535 A bekannt.

Ähnlich zeigt die DE 201 05 341 U1 eine Vorrichtung zum Desinfizieren und Entkeimen von Wasser sowie einen Dusch- oder Brausekopf mit einer derartigen Vorrichtung. Dabei handelt es sich um eine Durchflussvorrichtung, die im inneren ein längliches Rohr aus Quarzglas aufweist, in der eine ultraviolettes Licht abgegebene Lampe vorgesehen ist. Das Wasser strömt dabei in zwei Ringräumen hin- und wieder zurück, wobei die beiden Ringräume durch ein weiteres Quarzglasrohr in dem Strömungsweg getrennt sind, so dass das UV-Licht auf beide Strömungswege einwirken kann.

Weiter ist aus der US 2016/0331855 A1 ein Desinfektionssystem bekannt, bei dem eine UV-Lichtquelle in eine Wasserzapfstelle innenseitig oder außenseitig mit einem Durchstrahlungsfenster eingebaut bzw. die UV-Strahlung in die Wasserzapfstelle und damit in das darin geführte Wasser direkt eingekoppelt wird.

Nachteilig ist, dass bei den bekannten UV-Bestrahlungsanordnungen eine unmittelbare Desinfektion mit UV-Licht an der Wasserzapfstelle nur mit dort untergebrachten Bestrahlungselementen erreicht werden kann. Dies bedingt wiederum, dass die Energieversorgung elektrisch sicher und räumlich im Gehäuse des Brausekopfes und im Brausekopf auch die Elemente zur UV-Bestrahlung unterzubringen sind und vom Nutzer bei Verwendung der Handbrause auch getragen werden müssen. Allein die DE 101 57 355 A1 gibt Anregung, die UV-Strahlung im Bereich des Brauchwasseranschlusses über ein Anschlussstück in den flexiblen Schlauch der Handbrause einzukoppeln. Bei dieser Ausführung besteht jedoch der Nachteil darin, dass eine unmittelbare UV-Bestrahlung am Brausekopf nicht realisiert werden kann. Entsprechendes im Brausekopf befindliches Restwasser kann somit wiederum als Nährboden für die Vermehrung von Keimen, insbesondere Legionellen, dienen.

Aus der GB 2 288 974 A ist ein Verfahren und eine Vorrichtung zur Einleitung von Lichtstrahlen in Sanitärinstallationen, insbesondere Duschbrausen bekannt, bei der insbesondere sichtbares Licht, ggf. auch gefiltert, polarisiert oder maskiert in den von der Armatur ausgegebenen Wasserstrahl eingeleitet wird.

Ebenso betrifft die CN 103962256 B eine Duschvorrichtung, die Komponenten enthält, mit der unter anderem Lichteffekte in das abzugebende Wasser eingeleitet werden. Damit soll eine Phototherapie sowie auch eine Lichteffekt-, Magnet-, oder Musiktherapie ausgeführt werden können. Ferner kann die Duschvorrichtung auch eine ultraviolette Sterilisation und Luftreinigung enthalten.

Aus der WO 2011/052986 A2 ist ein Brausehalter bekannt, der an einer Wandfläche eines Badezimmers angeordnet ist. Der Brausehalter enthält eine UV-Lampe oder eine Ultraschallquelle zur Sterilisierung des in dem Brausehalter abgelegten Duschkopf. Dabei wirkt die Sterilisierungseinrichtung von außen auf die Wasseraustrittsöffnungen des Duschkopfes.

Die DE 296 14 998 U1 beschreibt eine Wasserentnahmearmatur mit einem Duschkopf und einer flexiblen Leitung, wobei in dem Duschkopf eine UV-Strahlungsabgabeeinrichtung vorgesehen ist, die über eine durch die flexible Leitung geführte Speiseleitung mit einer Stromversorgung verbunden ist.

Aufgabe der Erfindung ist es daher, ein Verfahren zur Desinfektion von Wasser mit einer flexibel an einem Schlauch angeschlossenen Wasserzapfstelle bzw. eine entsprechende Wasserzapfstellenanordnung anzugeben, bei der die Wasserzapfstelle, also insbesondere die Handbrause, weder die UV-Bestrahlungsmittel noch die entsprechende Energieversorgung beinhaltet, jedoch gleichwohl eine UV-Bestrahlung auch des in der Wasserzapfstelle befindlichen Restwassers ermöglicht.

Gelöst wird diese Aufgabe mit einem Verfahren gemäß Anspruch 1 und einer Wasserzapfstellenanordnung gemäß Anspruch 5.

Dadurch dass die UV-Bestrahlung von der Wasserzapfstellenhalterung auf die Wasserzapfstelle gerichtet wird, wenn die Wasserzapfstelle in der Wasserzapfstellenhalterung aufgenommen ist, wird die UV-Bestrahlung in der Wasserzapfstellenhalterung erzeugt und auf die Wasserzapfstelle gerichtet, sodass immer bei in der Wasserzapfstellenhalterung eingelegter Wasserzapfstelle eine UV-Bestrahlung und somit Desinfektion des in der Wasserzapfstelle enthaltenen Wassers, auch Restwassers möglich ist.

Vorrichtungsgemäß wird die Aufgabe dadurch gelöst, dass die Mittel zur UV-Bestrahlung in der Wasserzapfstellenhalterung angeordnet sind und in der Wasserzapfstelle beim Aufnehmen in der Wasserzapfstellenhalterung gegenüberliegend zum Mittel zur UV-Bestrahlung ein für UV-Strahlung durchlässiges Fenster angeordnet ist. Erfindungsgemäß kann somit die Wasserzapfstelle, insbesondere Handbrause, in der üblichen Weise gehandhabt werden, ohne dass die Wasserzapfstelle mit zusätzlichen elektrischen Einrichtungen gewichts- und volumenbelastet ist. In der Wasserzapfstelle ist lediglich an geeigneter Stelle ein UV-strahlungsdurchlässiges Fenster anzuordnen, das von dem entsprechenden UV-Bestrahlungsmittel an der Wasserzapfstellenhalterung bestrahlt werden kann. Insgesamt ist gewährleistet, dass durch die UV-Bestrahlung an der Wasserzapfstelle direkt vor dem Wasseraustritt einem Keimwachstum entgegengewirkt wird.

Wenn die UV-Bestrahlung nach dem Aufnehmen der Wasserzapfstelle in der Wasserzapfstellenhalterung eingeschaltet und für eine vorgegebene Einschaltdauer aufrechterhalten wird, wird nach der Verwendung der Wasserzapfstelle das in der Wasserzapfstelle verbleibende Restwasser durch die anschließende UV-Bestrahlung über einen vorgegebenen Zeitraum zuverlässig entkeimt.

Sollte kurze Zeit später die Wasserzapfstelle wieder in Betrieb genommen werden, beispielsweise bei einem unterbrochenen Duschgang oder bei einem zweiten unmittelbar folgenden Duschgang, kann zur Vermeidung von unnötiger zusätzlicher Entkeimung und damit zum Energiesparen vorgesehen sein, dass die UV-Bestrahlung beim nachfolgenden, erneuten Aufnehmen der Wasserzapfstelle in der Wasserzapfstellenhalterung nur eingeschaltet wird, wenn ein vorgegebenes erstes Wiederholungsintervall überschritten ist. Beispielsweise braucht eine Entkeimung nur alle 24 Stunden, 48 Stunden oder gar einmal wöchentlich stattfinden. Dies kann in Abhängigkeit von Erfahrungswerten oder Überprüfungen eingestellt werden.

Andererseits kann es von Vorteil sein, dass die UV-Bestrahlung bei Nichtgebrauch der Wasserzapfstelle eingeschaltet wird, wenn ein vorgegebenes zweites Wiederholungsintervall überschritten wird. Beispielsweise sollte eine UV-Bestrahlung zur Entkeimung des Restwassers bei einem längeren Zeitraum der Nichtbenutzung wiederholt werden, damit trotz der Entkeimung nach längerem Zuwarten die Keimzahl nicht zu groß wird. Beispielsweise kann so eine Entkeimung mindestens einmal wöchentlich oder nach einer Zeit von zwei oder vier Wochen als zweites Wiederholungsintervall erforderlich werden.

Vorrichtungsgemäß sind als geeignetes Mittel zur UV-Bestrahlung UVC emittierende LED-Halbleiter bevorzugt, da diese im Verhältnis zu Ihrer Strahlungsintensität einen niedrigen Stromverbrauch haben, schnell ihre gewünschte Strahlungsintensität erreichen und auf die optimale, keimtötende Lichtwellenlänge gut einstellbar sind.

Wenn die Mittel zur UV-Bestrahlung wasserdicht in der Wasserzapfstellenhalterung angeordnet sind, wobei eine UV-Licht durchlässige Abdeckung in der Wasserzapfstellenhalterung gegenüberliegend zum für UV-Strahlung durchlässigen Fenster der Wasserzapfstelle vorgesehen ist, ist auch die in der Wasserzapfstellenhalterung untergebrachte UV-Bestrahlungseinrichtung vor einer Beeinträchtigung durch eindringendes Wasser geschützt. Die UV-Bestrahlung wirkt quasi durch zwei UV-strahlungsdurchlässige Fenster, eins an der Wasserzapfstellenhalterung und eins in der Wasserzapfstelle, auf das in der Wasserzapfstelle verbleibende Restwasser gerichtet.

Vorteilhaft ist ferner, dass dem Mittel zur UV-Bestrahlung eine Stromversorgung mittels zugeführter Kleinspannung oder an der Wasserzapfstellenhalterung vorgesehener Batterie zugeordnet ist, womit die Energieversorgung allein an der Wasserzapfstellenhalterung, die fest an der Wand der Dusche oder des Bades angeordnet ist, vorzusehen ist. Zur Nachrüstung dieser Anordnung ist es dabei besonders bevorzugt, wenn die Mittel zur UV-Bestrahlung von einer in der Wasserzapfstellenhalterung vorgesehenen Batterie erfolgt, da somit keine zusätzlichen Installationsarbeiten zur Verlegung einer Niederspannungsleitung in den Bereich zur Wasserzapfstellenhalterung erforderlich ist. Dabei hat sich bei Versuchsmessungen herausgestellt, dass eine Versorgung der UV-Bestrahlungsmittel in der Wasserzapfstellenhalterung durchaus mit handelsüblichen 9 V-Block-Batterie für ca. 3.000 Desinfektionen ausreichend ist. Somit könnte eine solche Vorrichtung auch bei Batterieversorgung für mindestens zwei Jahre bei einer üblichen Nutzung in einem Privatbad ausreichen. Sowohl bei der energetischen Versorgung mit einer 9 V-Block-Batterie wie auch mit zugeführter Kleinspannung ist sichergestellt, dass die Bedienperson an der Wasserzapfstellenhalterung vor etwaigen gesundheitsgefährdenden elektrischen Stromschlägen geschützt ist.

Dadurch, dass an der Wasserzapfstellenhalterung ein Betätigungsmittel vorgesehen ist, das beim Aufnehmen der Wasserzapfstelle bei Nichtgebrauch an der Wasserzapfstellenhalterung die Mittel zur UV-Bestrahlung betätigt, wird bevorzugt die UV-Bestrahlung beim Wiedereinsetzen der Wasserzapfstelle, insbesondere Handbrause, in die Wasserzapfstellenhalterung ausgelöst. Gegebenenfalls kann vorgesehen werden, dass die UV-Bestrahlung erst nach einer Wartezeit von beispielsweise 10 Minuten einsetzt, damit die Bedienperson durch die eingeschaltete UV-Bestrahlung nicht irritiert oder gar beeinträchtigt wird.

Alternativ zur vorgenannten Ausgestaltung kann dadurch, dass die Wasserzapfstelle eine Handbrause mit Brausedüsen ist, wobei in der Handbrause vor den Brausedüsen Abschattungsmittel angeordnet sind, ein unbeabsichtigtes Austreten von UV-Licht vermieden werden. Diese Abschattungsmittel stellen nämlich sicher, dass in dem Duschkopf beim Duschen bzw. nach dem Benutzen des Duschkopfes keine ggf. gesundheitsschädliche UVC-Strahlung aus den Brausedüsen austreten kann. Die Abschattungsmittel verhindern einen unmittelbaren Strahlungskontakt von der UVC-Strahlungsquelle (LED-Halbleiter) zur Haut oder zu den Augen des Nutzers.

Ferner kann vorgesehen werden, dass in der Steuerungselektronik für die Mittel zur UV-Bestrahlung eine Sicherheitsschaltung vorgesehen ist, die die UV-Bestrahlung sofort beendet, sobald ein sicherheitsrelevanter Defekt festgestellt wird. Dies kann beispielsweise eine Zerstörung des Gehäuses der Wasserzapfstelle bzw. des Duschkopfes sein oder sonstige technische Defekte, die die Gefahr bergen, dass UVC-Strahlung ungehindert aus dem Bauteil austreten könnte. Als Sicherheitsschalter sind beispielsweise Lichtsensoren, Drucksensoren und/oder Feuchtesensoren denkbar. Gegebenenfalls ist eine Signalkommunikation zwischen Wasserzapfstelle und Wasserzapfstellenhalterung vorteilhaft.

Nachfolgend wird eine erfindungsgemäße Wasserzapfstellenanordnung anhand der beiliegenden Figuren detailliert beschrieben.Darin zeigt:
- Fig. 1: eine Wasserzapfstellenanordnung in einer Seitenansicht und
- Fig. 2: in einer Detailansicht ein Teil der Wasserzapfstelle.

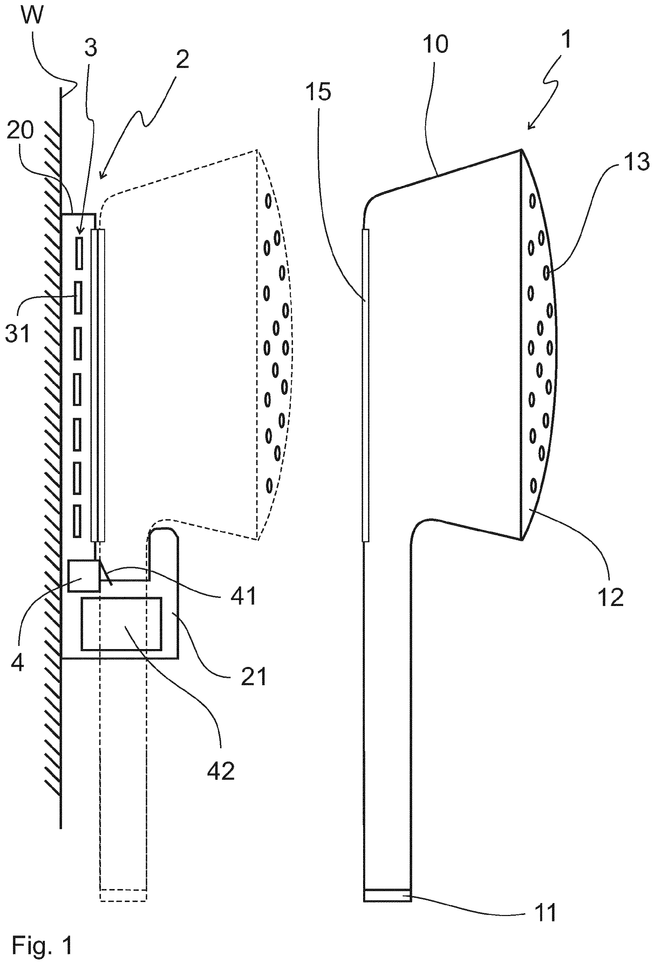

Fig. 1 zeigt eine Wasserzapfstelle 1 in Form einer Handbrause 1' sowie eine zugeordnete Wasserzapfstellenhalterung 2, die an einer Wand W befestigt ist. Die Handbrause 1' kann in die Wasserzapfstellenhalterung 2 in der in gestrichelter Linie dargestellter Weise abgelegt werden. Im Übrigen ist die Handbrause 1' in üblicher Weise frei handhabbar. Die Wasserzapfstelle 1, die als Handbrause bzw. Duschkopf 1' ausgebildet ist, ist an einem Duschschlauch zur Wasserzufuhr angeschlossen. Am stromabwärtigen Ende des Duschkopfes 1' ist als Wasserauslauf 12 eine Duschbrause mit einer Vielzahl von Brausedüsen 13 am Gehäuse 10 ausgebildet.

Die Wasserzapfstellenhalterung 2, die an der Wand W befestigt ist, weist ein Halterungsgehäuse 20 auf, an dem eine Aufnahme 21 zur Halterung des Duschkopfes bzw. der Handbrause 1' ausgebildet ist. Innerhalb des Halterungsgehäuses 20 der Wasserzapfstellenhalterung 2 ist ein Mittel zur UV-Bestrahlung 3 angeordnet. Das Mittel zur UV-Bestrahlung 3 weist eine Vielzahl von UVC emittierenden LEDs 31 auf, die beispielsweise auf einer Platine aufgebracht sind. Auf der Platine kann eine erforderliche Steuerungselektronik 4 angeordnet werden. In Fig. 1 ist die Steuerungselektronik 4 als Kästchen abgebildet. An der Steuerungselektronik 4 ist ein Betätigungsmittel 41 angeordnet, das beim Einhängen der Handbrause 1' in die Aufnahme 21 der Wasserzapfstellenhalterung 2 betätigt wird und ein Signal "Handbrause ordnungsgemäß an der Wasserzapfstellenhalterung 2 aufgenommen" meldet.

Die Stromversorgung erfolgt über eine Batterie 42, die ebenfalls im Halterungsgehäuse 20 der Wasserzapfstellenhalterung 2 untergebracht ist.

Um einerseits die elektrischen Komponenten in der Wasserzapfstellenhalterung 2 vor Feuchtigkeit zu schützen und gleichzeitig eine Abstrahlung der im Mittel zur UV-Bestrahlung 3 erzeugten UV-Strahlung zu erlauben, ist im Halterungsgehäuse 20 ein zweites für UV-Strahlung durchlässiges Fenster 22 vorgesehen. Ebenso ist im Gehäuse 10 der Handbrause 1' ein erstes für UV-Strahlung durchlässiges Fenster 15 angeordnet, das beim bestimmungsgemäßen Ablegen der Handbrause in der Wasserzapfstellenhalterung 2 genau an dem zweiten für UV-Strahlung durchlässigen Fenster 22 anliegt, sodass die in der Wasserzapfstellenhalterung 2 durch die Mittel zur UV-Bestrahlung 3 erzeugte UV-Strahlung in das Innere der Handbrause 1' einwirken kann.

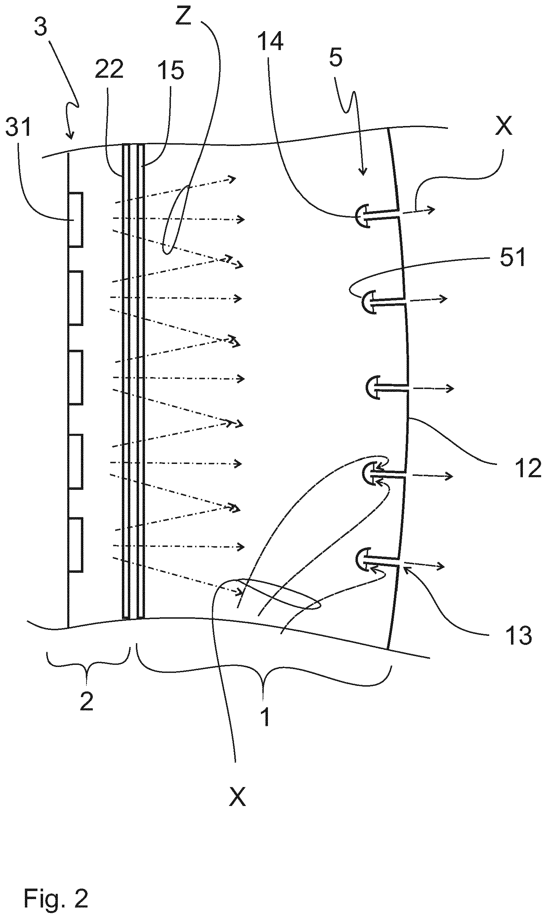

Diese Situation ist in Fig. 2 nochmals im Detail als Ausschnittdarstellung, teils geschnitten dargestellt. Die von den UVC emittierenden LED 31 abgegebene UVC-Strahlung Z der Mittel zur UV-Bestrahlung 3 wirken durch das zweite für UV-Strahlung durchlässige Fenster 22 und das parallel direkt anschließend angeordnete erste für UV-Bestrahlung durchlässige Fenster 15 auf den Innenraum der Handbrause 1'. Dort etwaig vorhandenes Restwasser wird durch die UVC-Strahlung desinfiziert.

Dabei weist der Duschkopf bzw. die Handbrause 1' in weiterer Ausgestaltung unmittelbar vor den Brausedüsen 13 Abschattungsmittel 5 auf. In einem Ausschnitt im Bereich der Duschbrause ist diese Situation in Fig. 2 dargestellt. Das Abschattungsmittel 5 besteht aus einer Haube 51, die innenseitig vor jeder Strömungseinlassöffnung 14 der Brausedüse 13 angeordnet ist. Diese UVCbeständige und lichtundurchlässige Haube 51 verhindert, dass von den LED-Halbleitern UVC-Licht durch die Brausedüse 13 in den Außenraum hindurchstrahlen könnte. Der Strömungseinlass 14 der Brausedüse 13 ist somit durch die Haube 51 sicher vor eindringender UVC-Strahlung Z geschützt. Da die Haube 51 beabstandet zum Strömungseinlass 14 der Brausedüse 13 angeordnet ist, kann das am Duschkopf 1 auszugebende Wasser entsprechend den in Fig. 2 dargestellten Strömungspfeilen X im Wesentlichen ungehindert durch die Brausedüse 13 fließen.

Nachfolgend wird die Funktionsweise des Wasserdesinfektionsverfahrens anhand der hier beschriebenen Wasserzapfstellenanordnung beschrieben.

Wenn ein Nutzer die Handbrause 1' benutzt hat und anschließend die Handbrause 1' in die Wasserzapfstellenhalterung 2 in die dafür vorgesehene Aufnahme 21 einsetzt, wird über das Betätigungsmittel 41 der Steuerungselektronik 4 gemeldet, dass eine gerade benutzte Handbrause 1' nunmehr abgelegt wurde. Die Steuerungselektronik 4 aktiviert nun die Mittel zur UV-Bestrahlung 3 durch Aufschalten der Spannungsversorgung aus Batterie 42 auf die UVC emittierenden LED 31. Entsprechend beginnt die UV- Bestrahlung des Innenraumes der Handbrause 1', da die UV-Strahlung, besonders bevorzugt UVC-Strahlung Z, vom Mittel zur UV-Bestrahlung 3 über die beiden Fenster 15, 22 in den Innenraum der Handbrause 1' einwirken.

Gegebenenfalls kann eine vorgegebene Wartezeit, von beispielsweise 10 Minuten, nach dem Einhängen der Handbrause 1' in die Aufnahme 21 der Wasserzapfstellenhalterung 2 voreingestellt werden, um eine Beeinträchtigung des Nutzers durch das UV-Licht zu vermeiden. Die Wartezeit wird ferner vorgegeben, um bei Duschpausen, beispielsweise für das Einseifen, nicht jedes Mal das Desinfektionsprogramm neu starten zu lassen. Wichtig ist nämlich insbesondere die Desinfektion für eine kommende längere Ruhezeit des Duschkopfes. Dabei wird die Steuerungselektronik 4 eine zur ausreichenden Desinfektion des in der Handbrause 1' befindlichen Restwassers erforderliche Einschaltdauer entsprechend vorgegebener Mindestwerte aufrechterhalten. Durch das Abtöten der im Restwasser noch vorhandenen Keime (Legionellen) wird verhindert, dass in dem Restwasser bei den dort herrschenden, optimalen Vermehrungsbedingungen die Keime sich stark vermehren könnten.

Ferner kann durch Vorsehen eines vorgegebenen ersten Wiederholungsintervalls erreicht werden, dass nicht jedes Mal nach Verwendung der Handbrause erneut eine Wasserdesinfektion gestartet wird. Das erste Wiederholungsintervall kann beispielsweise 24 Stunden, 48 Stunden oder gar eine ganze Woche umfassen. Damit kann sichergestellt werden, dass eine Desinfektion nur beispielsweise binnen 24 Stunden einmal durchgeführt wird, unabhängig von der Häufigkeit der Benutzung der Dusche und des Ablegens der Handbrause 1' in der Wasserzapfstellenhalterung 2.

Darüber hinaus kann noch ein zweites Wiederholungsintervall in der Steuerungselektronik 4 hinterlegt sein, der dafür sorgt, dass bei einer längeren Nichtbenutzung der Dusche etwaiges in der Handbrause 1' befindliches Restwasser mit darin doch noch enthaltenen Keimen zu einer erneuten starken Vermehrung führen. Dies kann beispielsweise bei einer längeren Nichtbenutzung von beispielsweise zwei Wochen oder vier Wochen für das zweite Wiederholungsintervall voreingestellt sein. Dies würde bedeuten, dass nach beispielsweise vier Wochen Nichtbenutzung der Handbrause 1' automatisch eine Desinfektion abläuft.

### Bezugszeichenliste

- 1: Wasserzapfstelle
- 1': Duschkopf, Handbrause
- 10: Gehäuse
- 11: Zulaufanschluss
- 12: Wasserauslauf
- 13: Brausedüse
- 14: Strömungseinlassöffnung
- 15: erstes Fenster

- 2: Wasserzapfstellenhalterung
- 20: Halterungsgehäuse
- 21: Aufnahme
- 22: zweites Fenster

- 3: Mittel zur UV-Bestrahlung
- 31: UVC emittierende LED

- 4: Steuerungselektronik
- 41: Betätigungsmittel
- 42: Batterie

- 5: Abschattungsmittel
- 51: Haube

- W: Wand
- X: Wasserströmungsrichtung
- Z: UVC-Strahlung

## Patentansprüche

1. Verfahren zur Desinfektion von Wasser mittels UV-Bestrahlung in Wasserversorgungsanlagen in Gebäuden, Schiffen oder dergleichen mit einer flexibel an einem Schlauch angeschlossenen Wasserzapfstelle (1), nämlich Handbrause (1'), und einer Wasserzapfstellenhalterung (2) zum Aufnehmen (21) der Wasserzapfstelle (1) bei Nichtgebrauch, wobei die UV-Bestrahlung an der Wasserzapfstelle (1) erfolgt, **dadurch gekennzeichnet, dass** die UV-Bestrahlung von der Wasserzapfstellenhalterung (2) auf ein für UV-Strahlung durchlässiges Fenster der Wasserzapfstelle (1) gerichtet wird, wenn die Wasserzapfstelle (1) in der Wasserzapfstellenhalterung (2) aufgenommen ist.

2. Verfahren nach Anspruch 1, **dadurch gekennzeichnet, dass** die UV-Bestrahlung nach dem Aufnehmen (21) der Wasserzapfstelle (1) in der Wasserzapfstellenhalterung (2) eingeschaltet und für eine vorgegebene Einschaltdauer aufrechterhalten wird.

3. Verfahren nach Anspruch 2, **dadurch gekennzeichnet, dass** die UV-Bestrahlung beim nachfolgenden, erneuten Aufnehmen (21) der Wasserzapfstelle (1) in der Wasserzapfstellenhalterung (2) nur eingeschaltet wird, wenn ein vorgegebenes erstes Wiederholungsintervall überschritten ist.

4. Verfahren nach Anspruch 2 oder 3, **dadurch gekennzeichnet, dass** die UV-Bestrahlung bei Nichtgebrauch der Wasserzapfstelle (1) eingeschaltet wird, wenn ein vorgegebenes zweites Wiederholungsintervall überschritten wird.

5. Wasserzapfstellenanordnung für Wasserversorgungsanlagen in Gebäuden, Schiffen oder dergleichen mit einer flexibel an einem Schlauch angeschlossenen Wasserzapfstelle (1), nämlich Handbrause (1'), und einer Wasserzapfstellenhalterung (2) für das Aufnehmen (21) der Wasserzapfstelle (1) bei Nichtgebrauch, wobei Mittel zur UV-Bestrahlung (3) des in der Wasserzapfstelle (1) geführten Wassers vorgesehen sind, **dadurch gekennzeichnet, dass** die Mittel zur UV-Bestrahlung (3) in der Wasserzapfstellenhalterung (2) angeordnet sind und in der Wasserzapfstelle (1) beim Aufnehmen (21) in der Wasserzapfstellenhalterung (2) gegenüberliegend zum Mittel zur UV-Bestrahlung (3) ein erstes, für UV-Strahlung durchlässiges Fenster (15) angeordnet ist.

6. Wasserzapfstellenanordnung nach Anspruch 5, **dadurch gekennzeichnet, dass** die Mittel zur UV-Bestrahlung (3) UVC emittierende LED-Halbleiter (31) sind.

7. Wasserzapfstellenanordnung nach Anspruch 5 oder 6, **dadurch gekennzeichnet, dass** die Mittel zur UV-Bestrahlung (3) wasserdicht in der Wasserzapfstellenhalterung (2) angeordnet sind, wobei ein zweites für UV-Licht durchlässiges Fenster (22) in der Wasserzapfstellenhalterung (2) gegenüberliegend zum ersten, für UV-Strahlung durchlässigen Fenster (15) der Wasserzapfstelle (1) vorgesehen ist.

8. Wasserzapfstellenanordnung nach Anspruch 5, 6 oder 7, **dadurch gekennzeichnet, dass** dem Mittel zur UV-Bestrahlung (3) eine Stromversorgung mittels zugeführter Kleinspannung oder an der Wasserzapfstellenhalterung (2) vorgesehener Batterie (42) zugeordnet ist.

9. Wasserzapfstellenanordnung nach einem der Ansprüche 5 bis 8, **dadurch gekennzeichnet, dass** an der Wasserzapfstellenhalterung (2) ein Betätigungsmittel (41) vorgesehen ist, das beim Aufnehmen (21) der Wasserzapfstelle (1) bei Nichtgebrauch an der Wasserzapfstellenhalterung (2) die Mittel zur UV-Bestrahlung (3) betätigt.

10. Wasserzapfstellenanordnung nach einem der Ansprüche 5 bis 9, **dadurch gekennzeichnet, dass** die Wasserzapfstelle (1) eine Handbrause mit Brausedüsen (13) ist, wobei in der Handbrause (1') vor den Brausedüsen (13) Abschattungsmittel (5) angeordnet sind.

## Claims

1. A method of disinfecting water by means of UV irradiation in water supply installations in buildings, ships or the like, having a water tapping point (1), namely a hand-held spray (1'), flexibly attached to a hose, and a water tapping point holding means (2) to receive (21) the water tapping point (1) when not in use, wherein the UV irradiation takes place at the water tapping point (1), **characterised in that** the UV irradiation is directed from the water tapping point holding means (2) onto a window, permeable to UV radiation, of the water tapping point (1) when the water tapping point (1) is received in the water tapping point holding means (2).

2. A method according to claim 1, **characterised in that** the UV irradiation is switched on after the water tapping point (1) has been received (21) in the water tapping point holding means (2) and is maintained for a predetermined switch-on time.

3. A method according to claim 2, **characterised in that** upon subsequent receiving (21) of the water tapping point (1) in the water tapping point holding means (2) once again, the UV irradiation is only switched on when a predetermined first repetition time interval is exceeded.

4. A method according to claim 2 or 3, **characterised in that** when the water tapping point (1) is not in use, the UV irradiation is switched on when a predetermined second repetition time interval is exceeded.

5. A water tapping point arrangement for water supply installations in buildings, ships or the like, having a water tapping point (1), namely a hand-held spray (1'), flexibly attached to a hose, and a water tapping point holding means (2) to receive (21) the water tapping point (1) when not in use, wherein there are provided means (3) for UV irradiation of the water guided in the water tapping point (1), **characterised in that** the means (3) for UV irradiation are arranged in the water tapping point holding means (2) and a first window (15), permeable to UV radiation, is arranged in the water tapping point (1) such that it is opposite the means (3) for UV radiation upon receiving (21) in the water tapping point holding means (2).

6. A water tapping point arrangement according to claim 5, **characterised in that** the means (3) for UV irradiation are LED semiconductors (31) emitting UVC.

7. A water tapping point arrangement according to claim 5 or 6, **characterised in that** the means (3) for UV irradiation are arranged in the water tapping point holding means (2) in a water-tight manner, wherein a second window (22), permeable to UV light, is provided in the water tapping point holding means (2) such that it is opposite the first window (15), permeable to UV radiation, of the water tapping point (1).

8. A water tapping point arrangement according to claim 5, 6 or 7, **characterised in that** a power supply by means of fed low voltage or a battery (42) provided at the water tapping point holding means (2) is associated with the means (3) for UV irradiation.

9. A water tapping point arrangement according to any one of claims 5 to 8, **characterised in that** an actuating means (41) is provided at the water tapping point holding means (2) and actuates the means (3) for UV irradiation upon receiving (21) of the water tapping point (1) at the water tapping point holding means (2) when not in use.

10. A water tapping point arrangement according to any one of claims 5 to 9, **characterised in that** the water tapping point (1) is a hand-held spray with spray nozzles (13), wherein screening means (5) are arranged in the hand-held spray (1'), in front of the spray nozzles (13).

## Revendications

1. Procédé de désinfection de l'eau par rayonnement UV dans des installations d'alimentation en eau de bâtiments, de navires ou d'infrastructures analogues, munies d'une prise d'eau (1) raccordée de manière flexible à un tuyau, à savoir d'une douchette (1'), et d'un support de prise d'eau (2) pour recevoir (21) la prise d'eau (1) quand elle n'est pas utilisée, l'irradiation UV étant réalisée au niveau de la prise d'eau (1), **caractérisée en ce que** l'irradiation UV est dirigée, lorsque la prise d'eau (1) est reçue dans le support de prise d'eau (2), depuis le support de prise d'eau (2) vers une fenêtre transparente au rayonnement UV de la prise d'eau (1).

2. Procédé selon la revendication 1, **caractérisé en ce que** l'irradiation UV est activée après réception (21) de la prise d'eau (1) dans le support de prise d'eau (2) et est maintenue pendant une durée d'activation prédéterminée.

3. Procédé selon la revendication 2, **caractérisé en ce qu'**après une nouvelle réception (21) suivante de la prise d'eau (1) dans le support de prise d'eau (2), l'irradiation UV n'est activée qu'après qu'un premier intervalle de répétition prédéterminé est écoulé.

4. Procédé selon la revendication 2 ou 3, **caractérisé en ce qu'**en cas de non-utilisation de la prise d'eau (1), l'irradiation UV est activée après qu'un deuxième intervalle de répétition prédéterminé est écoulé.

5. Agencement de prise d'eau pour des installations d'alimentation en eau de bâtiments, de navires ou d'infrastructures analogues, munies d'une prise d'eau (1) raccordée de manière flexible à un tuyau, à savoir d'une douchette (1'), et d'un support de prise d'eau (2) pour recevoir (21) la prise d'eau (1) quand elle n'est pas utilisée, des moyens d'irradiation UV (3) de l'eau acheminée dans la prise d'eau (1) étant prévus, **caractérisé en ce que** les moyens d'irradiation UV (3) sont disposés dans le support de prise d'eau (2) et **en ce qu'**une première fenêtre (15) transparente au rayonnement UV est disposée dans la prise d'eau (1), à l'opposée des moyens d'irradiation UV (3) lors de la réception (21) dans le support de prise d'eau (2).

6. Agencement de prise d'eau selon la revendication 5, **caractérisé en ce que** les moyens d'irradiation UV (3) sont des semi-conducteurs DEL à émission UVC (31).

7. Agencement de prise d'eau selon la revendication 5 ou 6, **caractérisé en ce que** les moyens d'irradiation UV (3) sont disposés de manière étanche à l'eau dans le support de prise d'eau (2), une deuxième fenêtre (22) transparente à la lumière UV étant prévue dans le support de prise d'eau (2) à l'opposé de la première fenêtre transparente au rayonnement UV (15) de la prise d'eau (1).

8. Agencement de prise d'eau selon la revendication 5, 6 ou 7, **caractérisé en ce qu'**une alimentation en électricité est attribuée aux moyens d'irradiation UV (3) au moyen d'une basse tension d'alimentation ou d'une batterie (42) prévue sur le support de prise d'eau (2).

9. Agencement de prise d'eau selon l'une des revendications 5 à 8, **caractérisé en ce qu'**un moyen d'actionnement (41) est prévu sur le support de prise d'eau (2), lequel moyen actionne les moyens d'irradiation UV (3) du support de prise d'eau (2) quand la prise d'eau (1) est reçue (21) lorsqu'elle n'est pas utilisée.

10. Agencement de prise d'eau selon l'une des revendications 5 à 9, **caractérisé en ce que** la prise d'eau (1) est une douchette avec des buses de douche (13), des moyens occultants (5) étant disposés dans la douchette (1') devant les buses de douche (13).
